(19) Europäisches Patentamt
European Patent Office
Office européen des brevets

(11) Numéro de publication: **0 318 400 A1**

(12) **DEMANDE DE BREVET EUROPEEN**

(21) Numéro de dépôt: **88420366.2**

(22) Date de dépôt: **27.10.88**

(51) Int. Cl.4: **C 07 D 249/08**
C 07 D 233/60, A 01 N 43/50,
A 01 N 43/653

(30) Priorité: **29.10.87 FR 8715246**

(43) Date de publication de la demande:
**31.05.89 Bulletin 89/22**

(84) Etats contractants désignés:
**AT BE CH DE ES FR GB GR IT LI LU NL SE**

(71) Demandeur: **RHONE-POULENC AGROCHIMIE**
**14-20, rue Pierre Baizet**
**F-69009 Lyon (FR)**

(72) Inventeur: **Greiner, Alfred**
**35 Route de St. Romain**
**F-69450 St.-Cyr-au-Mont-d'Or (FR)**

**Pepin, Régis**
**27 Montée Castellane**
**F-69140 Rilleux-la-Pape (FR)**

(74) Mandataire: **Ranguis, Patrick Frédéric et al**
**RHONE POULENC AGROCHIMIE Service DPI B.P. 9163**
**F-69263 Lyon Cédex 09 (FR)**

(54) **Composés à groupements triazole, utilisation de ceux-ci à titre de fongicides et procédés de préparation.**

(57) L'invention concerne des composés de formule :

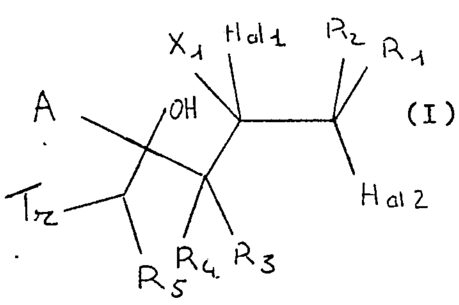

(I)

A est le groupement (Y)nPh-(CH2)m oùPh est un noyau phényle et m=0 ou 1, de préférence 0,
Y est un atome d'halogène ou un groupe cyano ou nitro, ou un groupe alkyle ou alkoxy éventuellement halogéné, n est un nombre entier positif ou nul, inférieur à 6,
Tr représente un groupe 1,2,4-triazole 1-yl,
$R_1$ à $R_5$ représentent l'atome d'hydrogène ou un radical alkyle inférieur,
$X_1$, represénte un atome d'hydrogène ou un radical hydrocarbyle.
Ha11, Ha12, identiques ou différents, représentent un atome d'halogène, de préférence choisis parmi le chlore ou le brome et avantageusement Ha11 et Ha12 sont identiques.
Utilisation de ces composés à titre de fongicides .

EP 0 318 400 A1

## Description

### COMPOSES A GROUPEMENTS TRIAZOLE, UTILISATION DE CEUX-CI A TITRE DE FONGICIDES ET PROCEDES DE PREPARATION

La présente invention concerne de nouveaux composés, à usage phytosanitaire, à groupements triazole ou imidazole. Elle concerne également les procédés de préparation desdits composés. Elle concerne ensuite l'utilisation à titre de fongicides de ces composés, les compositions fongicides à base de ces composés et les procédés pour lutter contre les maladies fongiques des cultures utilisant ces composés.

De nombreux produits à groupes triazole, notamment des fongicides, sont déjà connus en particulier par la demande de brevet européen EP-A-0047594.

Un but de la présente invention est de proposer des composés présentant des propriétés améliorées dans le traitement des maladies fongiques. En particulier, ces composés présentent des propriétés améliorées dans le traitement des piricularioses, des oidiums et des rouilles, notamment des céréales.

Ceux-ci sont caractérisés en ce qu'ils répondent à la formule I indiquée en fin de description dans laquelle:
A est le groupement (Y)nPh-(CH2)m où Ph est un noyau phényle et m=0 ou 1, de préférence 0,
Y est un atome d'halogène ou un groupe cyano ou nitro, ou un groupe alkyle ou alkoxy, phényle, phénoxy, benzyle, benzyloxy, ces groupes étant éventuellement halogénés,
n est un nombre entier positif ou nul, inférieur à 6, les groupements Y pouvant être identiques ou différents lorsque n est plus grand que 1,
Tr représente un groupe 1,2,4-triazole 1-yl, Im représente un groupe 1,3-imidazole 1-yl
$R_1$ à $R_5$, identiques ou différents, représentent l'atome d'hydrogène ou un radical alkyle inférieur, cycloalkyle inférieur, aryle (notamment phényle), aralkyle (notamment benzyle), alkoxy inférieur, alcanoyle inférieur, aroyle (notamment benzoyle), ces divers radicaux pouvant être éventuellement substitués (par exemple par un ou plusieurs atomes ou radicaux tels que les atomes d'halogènes, et les radicaux alkoxy inférieurs),
$X_1$ représente l'atome d'hydrogène, un atome d'halogène, un radical alkyle inférieur, un radical cycloalkyle inférieur, un radical aryle (notamment phényle), ou aralkyle (notamment benzyle), ces divers radicaux pouvant être éventuellement substitués de façon similaires aux groupes $R_1$ à $R_5$,
un groupement Q-$R_6$ dans lequel Q représente 0 ou S, et $R_6$ représente l'atome d'hydrogène ou un radical alkyle inférieur, un radical cycloalkyle inférieur, aryle (notamment phényle), aralkyle (notamment benzyle), acyle ou thioacyle (notamment acétyle, thioacétyle, propionyle, thiopropionyle), alkyloxythioyle, aryloxythioyle ou aralkyloxythioyle (le terme thioyle correspond à C = S (S)), ces radicaux pouvant être substitués de façon similaire aux groupes $R_1$ à $R_5$,
un groupement -N $R_8R_9$ dans lequel $R_8$ et $R_9$, identiques ou différents, représentent l'atome d'hydrogène ou un radical alkyle inférieur, un radical cycloalkyle inférieur, aryle (notamment phényle), aralkyle (notamment benzyle), ces divers radicaux pouvant être éventuellement substitués de façon similaire aux groupes $R_1$ à $R_5$, ou bien $R_8$ et $R_9$ peuvent former ensemble un radical unique divalent hydrocarboné, comprenant de 3 à 6 atomes de carbones, l'un de ces atomes de carbone pouvant être remplacé par un atome d'oxygène, de soufre ou d'azote, ledit radical unique divalent étant lui-même éventuellement substitué (par exemple par un ou plusieurs atomes d'halogène ou radicaux alkyle inférieur, alkoxy inférieur éventuellement halogénés, hydroxy),
Ha11, Ha12, identiques ou différents, représentent un atome d'halogène, de préférence choisis parmi le chlore ou le brome et avantageusement Ha11 et Ha12 sont identiques.

L'invention concerne également les formes salifiées des composés selon l'invention. Les formes salifiées sont les formes acceptables en agriculture parmi lesquelles on peut citer : les chlorhydrate, sulfate, oxalate, nitrate ou arylsulfonate ainsi que les complexes d'addition de ces composés avec des sels métalliques, et notamment des sels de fer, chrome, cuivre, manganèse, zinc, cobalt, étain, magnésium et aluminium.

A titre d'exemple, des complexes avec le zinc peuvent être obtenus en faisant réagir le composé de formule I avec le chlorure de zinc.

Au sens du présent texte, on entend que l'adjectif inférieur, lorsqu'il qualifie un radical organique, signifie que ce radical comporte au plus six atomes de carbone. Ce radical peut être linéaire ou ramifié.

Le déposant souhaite souligner que les planches annexes ne sauraient en aucune manière être considérées comme des dessins mais comme faisant partie intégrante de la description de l'invention.

Les composés de formule I et les composés éventuellement utilisables à titre d'intermédiaires dans les procédés de préparation et qui seront définis à l'occasion de la description de ces procédés peuvent exister sous une ou plusieurs formes d'isomères selon le nombre de centres asymétriques de la molécule. L'invention concerne donc aussi bien tous les isomères optiques que leurs mélanges racémiques et les diastéréoisomères correspondants. La séparation des diastéréoisomères et/ou des isomères optiques peut s'effectuer selon les méthodes connues en soi.

En vue des applications fongicides il a été trouvé que l'invention concernait de préférence les composés de formule I dans laquelle Y est un halogène et n = 1, 2 ou 3.

Il a été également trouvé qu'il était préférable d'utiliser les composés de formule I dans laquelle n = 1 ou 2, et Y est un atome d'halogène placé en ortho et/ou en para.

De préférence encore n = 2 et Y est un atome d'halogène, avantageusement le chlore placé en ortho et en para.

Les composés de formule I préférés sont ceux dont les groupements $R_1$ à $R_5$, $X_1$ correspondent à un atome d'hydrogène, à un groupement alkyle inférieur et/ou Ha11 et Ha12 correspondent tous les deux à l'atome de chlore.

De préférence encore, les groupements $R_1$ à $R_5$, $X_1$ correspondent à un atome d'hydrogène.

On préférera les composé à groupe triazole.

La présente invention concerne également des procédés de préparation des composés selon l'invention.

Etape a)

On fait réagir une halogènocétone de formule IIa obtenue par un procédé connu, dans laquelle A, $R_5$ ont la même signification que celle donnée pour les composés de formule I et Z correspond à un atome d'halogène, avec un organométallique de formule IIb, dans laquelle $R_1$ à $R_4$, $X_1$, ont la même signification que ci-dessus et M correspond à un métal alcalin ou à un magnésien (Mg Hal) ou à un zincique (Zn Hal) par exemple, dans un solvant, choisi de préférence parmi les éthers, tels que l'éther diéthylique ou le tétrahydrofuranne, les hydrocarbures aliphatiques, alicycliques ou aromatiques tels que l'hexane, le toluène, à une température choisie entre -50° C et le reflux du solvant considéré et dans un rapport molaire II a : II b compris de préférence entre 1,1 et 0,2. La réaction conduit au composé de formule IIc après neutralisation du milieu réactionnel. Ce procédé est décrit dans EP-A-0033501.

Etape b)

On fait réagir ensuite le composé de formule IIc avec un imidazole ou un triazole non substitué en présence de base organique ou minérale par exemple la pyridine, la triéthylamine, la soude, la potasse, les carbonates et bicarbonates de métaux alcalins ou alcalino terreux et dans un solvant approprié tel que par exemple les alcools, les cétones, les amides, les nitriles, les hydrocarbures aromatiques éventuellement halogénés à une température comprise entre 80° et le reflux du solvant et dans un rapport molaire IIc : imidazole ou triazole de préférence compris entre 1,1 et 0,2, ce qui conduit au composé de formule IId. La réaction passe en général par un intermédiaire époxyde de formule IIe qui peut éventuellement être isolé ou préparé séparémment par des méthodes connues de l'homme de l'art. Par exemple en faisant réagir le composé IIc avec une base citée ci-dessus. Voir par exemple le procédé décrit dans EP-A-0097425.

En tous les cas l'étape de greffage est avantageusement effectuée en présence d'un accepteur d'acide, en milieu anhydre ou non anhydre, dans un solvant, inerte dans les conditions de la réaction, généralement entre 50 et 180° C et de préférence à une température voisine de la température d'ébullition du solvant. Comme accepteurs d'acide, on peut mentionner des bases minérales comme par exemple la soude ou la potasse, les carbonates de métaux alcalins ou de métaux alcalinoterreux, des bases azotées comme la triéthylamine, des amines quaternaires comme l' hydroxyde de tétrabutylammonium, ou des hydroxydes de phosphonium. Comme solvants on utilise avantageusement des solvants aprotiques polaires, tels que par exemple, le diméthylformamide, le diméthylacétamide, le diméthylsulfoxyde, l'acétone, la méthyl-éthyl-cétone, l'acétonitrile, la N-méthylpyrrolidone ou des solvants protiques polaires tels que le méthanol ou le propanol. Si désiré, cette réaction peut être effectuée en présence d'un catalyseur approprié. Comme catalyseur utilisable, on peut mentionner des catalyseurs de transfert de phases, tels que par exemple, des dérivés d'ammonium quaternaires comme le chlorure de tétrabutylammonium.

La réaction est de préférence effectuée en excès molaire de dérivé de triazole ou imidazole de préférence compris entre 1,05 et 1,5.

Lorsque l'on utilise un solvant, on préférera travailler dans un milieu dilué comportant 1 % à 70 % en poids de composé de formule IIc, IIe par rapport à la solution totale.

L'accepteur d'acide est au moins présent en quantité stoechiométrique en équivalent d'atome d'hydrogène labile du triazole ou imidazole. En général un rapport en équivalent molaire compris entre 1 et 2,5 sera satisfaisant.

Il est évident que le dérivé salifié du triazole ou imidazole peut être préparé à part et dans ce cas la présence d'un accepteur d'acide n'est pas requise pour la réaction de greffage. Cette préparation s'effectue en milieu anhydre ou non anhydre dans un solvant dans les mêmes conditions opératoires que celles décrites dans le cas de la formation in situ du dérivé triazole ou imidazole salifié.

Etape c)

On additionne sur le composé IId de préférence mole à mole une molécule d'halogène ou d'halogène mixte dans un solvant inerte tel que les hydrocarbures saturés ou aromatiques éventuellement halogénés, ce qui conduit au composé I. On peut également effectuer cette addition en milieu acide minéral concentré ce qui a pour avantage de faire précipiter sous forme de sel correspondant le composé I

La présente invention concerne également l'utilisation des composés de formule I à titre de fongicides.

Les composés selon l'invention peuvent être utilisés pour la lutte tant préventive que curative contre les champignons, notamment de type basidiomycètes, ascomycètes, adelomycètes ou fungi-imperfecti, en particulier les rouilles, oïdium, piétin-verse, fusarioses, helminthosporioses, septorioses, rhizoctones des végétaux et des plantes en général et en particulier des céréales telles que le blé, l'orge, le seigle, l'avoine et leurs hybrides et aussi le riz et le maïs. Les composés selon l'invention sont actifs en particulier contre les champignons notamment de type basidiomycètes, ascomycètes, adelomycètes ou fungi-imperfecti comme Botrytis cinerea, Erysiphe graminis, Puccinia recondita, Piricularia oryzae, Cercospora beticola, Puccinia

striiformis, Erysiphe cichoracearum, Fusarium oxysporum (melonis), Pyrenophora avenae, Septoria nodorum, Septoria tritici, Venturia inaequalis, Whetzelinia sclerotiorum, Monilia laxa, Mycoaphaerella fijiensis, Marssonina panattoniana, Alternaria solani, Aspergillus niger, Cercospora arachidicola, Cladosporium herbarum, Helminthosporium oryzae, Penicillium expansum, Pestalozzia sp, Phialophora cinerescens, Phoma betae, Phoma foveata, Phoma lingam, Ustilago maydis, Verticillium dahliae, Ascochyta pisi, Guignardia bidwellii, Corticium rolfsii, Phomopsis viticola, Sclerotinia sclerotiorum, Sclerotinia minor, Coryneum cardinale, Rhizoctonia solani.

Ils sont aussi et encore actifs contre les champignons suivants : Acrostalagmus koningi, les Alternaria, les Colletotrichum, Corticium rolfsii, Diplodia natalensis, Gaeumannomyces graminis, Gibberella fujikuroi, Hormodendron cladosporioides, Lentinus degener ou tigrinus, Lenzites quercina, Memnoniella echinata, Myrothecium verrucaria, Paecylomyces varioti, Pellicularia sasakii, Phellinus megaloporus, Polystictus sanguineus, Poria vaporaria, Sclerotium rolfsii, Stachybotris atra, les Stereum, Stilbum sp. Trametes trabea, Trichoderma pseudokoningi, Trichothecium roseum.

Les composés de l'invention sont spécialement intéressants par leur spectre large au niveau des maladies des céréales (oïdium, rouille) et du riz (piriculariose). Ils présentent également un grand intérêt en raison de leur activité sur la pourriture grise (Botrytis) et les cercosporioses, et, de ce fait, ils peuvent être appliqués sur des cultures aussi variées que la vigne, les cultures maraîchères et l'arboriculture et les cultures tropicales telles que l'arachide, le bananier, le caféier, la noix de pécan et d'autres.

Les composés peuvent également être utilisés dans le traitement des semences (céréales, cotonnier, betterave, colza, graines fourragères) par exemple sous la forme d'enrobage ou de pélliculage. Ainsi, on pourra trouver dans US 3,989,501, col 7, 1 17-23, une forme d'application. De même, dans FR-A-2 588 442. On pourra également utiliser des flo aqueux.

En général ces formulations sont déja connues voir par exemple "catalogue of pesticide formulation types and international coding system" édité par le GIFAP technical monograph n°2, pages 12 à 14, révisée en janvier 1984.

Outre les applications déjà décrites plus haut, les produits selon l'invention présentent en outre une excellente activité biocide à l'égard de nombreuses autres variétés de microorganismes parmi lesquelles on peut citer à titre non limitatif, des champignons comme ceux des genres :

- Pullularia comme l'espèce P. pullulans,
- Chaetomium comme l'espèce C. globosum,
- Aspergillus comme l'espèce Aspergillus niger,
- Coniophora comme l'espèce C. puteana.

En raison de leur activité biocide, les produits de l'invention permettent de combattre efficacement les microorganismes dont la prolifération crée de nombreux problèmes dans les domaines agricole et industriel. A cet effet, ils conviennent tout spécialement bien à la protection des végétaux ou de produits industriels tels que le bois, le cuir, les peintures, le papier, les cordages, les plastiques, les circuits d'eau industriels.

Ils sont tout particulièrement bien adaptés à la protection des produits lignocellulosiques et notamment du bois, qu'il s'agisse de bois d'ameublement, de charpente ou de bois exposé aux intempéries tels que les bois de clôture, les piquets de vignes, les traverses de chemin de fer.

Les composés selon l'invention utilisés seuls ou sous la forme de compositiona telles que définies ci-aprés dans les traitements du bois, sont généralement mis en oeuvre avec des solvants organiques et peuvent être éventuellement associés à un ou plusieurs produits biocides connus tels que le pentachlorophénol, les sels métalliques, notamment de cuivre, de manganèse, de cobalt, de chrome, de zinc dérivés d'acides minéraux ou carboxyliques (acides heptanoïque, octanoïque, naphténique); les complexes organiques de l'étain, le mercaptobenzothiazole, les insecticides tela que les pyrethroïdes ou les organochlorés.

Ils présentent enfin une excellente sélectivité vis-à-vis des cultures.

Ils s'appliquent avantageusement à des doses de 0,005 à 5 kg/ha, et plus spécifiquement de 0,01 à 0,5 kg/ha.

Pour leur emploi pratique, les composés selon l'invention sont rarement utilisés seuls. Le plus souvent ils font partie de compositions. Ces compositions, utilisables pour la protection des végétaux contre les maladies fongiques, ou dans les compositions régulatrices de la croissance des plantes, contiennent comme matière active un composé selon l'invention tel que décrit précédemment en association avec les supports solides ou liquides, acceptables en agriculture et/ou les agents tensio-actifs également acceptables en agriculture. En particulier sont utilisables les supports inertes et usuels et les agents tensio-actifs usuels.

Par le terme "support", dans le présent exposé, on désigne une matière organique ou minérale, naturelle ou synthétique, avec laquelle la matière active est associée pour faciliter son application sur la plante, sur des graines ou sur le sol. Ce support est donc généralement inerte et il doit être acceptable en agriculture, notamment sur la plante traitée. Le support peut être solide (argiles, silicates naturels ou synthétiques, silice, résines, cires, engrais solides, etc...) ou liquide (eau, alcools, cétones, fractions de pétrole, hydrocarbures aromatiques ou paraffiniques, hydrocarbures chlorés, gaz liquéfiés, etc...).

L'agent tensioactif peut être un agent émulsionnant, dispersant ou mouillant de type ionique ou non ionique. On peut citer par exemple des sels d'acides polyacryliques, des sels d'acides lignosulfoniques, des sels d'acides phénolsulfoniques ou naphtalènesulfoniques, des polycondensats d'oxyde d'éthylène sur des alcools gras ou sur des acides gras ou sur des amines grasses, des phénols substitués (notamment des alkylphénols ou des arylphénols), des sels d'esters d'acides sulfosucciniques, des dérivés de la taurine

(notamment des alkyltaurates), des esters phosphoriques d'alcools ou de phénols polyoxyéthylés. La présence d'au moins un agent tensioactif est généralement indispensable lorsque la matière active et/ou le support inerte ne sont pas solubles dans l'eau et que l'agent vecteur de l'application est l'eau.

Pour leur application, les composés de formule (I) se trouvent donc généralement sous forme de compositions ; ces compositions selon l'invention sont elles-mêmes sous des formes assez diverses, solides ou liquides.

Comme formes de compositions solides, on peut citer les poudres pour poudrage ou dispersion (à teneur en composé de formule (I) pouvant aller jusqu'à 100 %) et les granulés, notamment ceux obtenus par extrusion, par compactage, par imprégnation d'un support granulé, par granulation à partir d'une poudre (la teneur en composé de formule (I) dans ces granulés étant entre 1 et 80 % pour ces derniers cas).

Comme formes de compositions liquides ou destinées à constituer des compositions liquides lors de l'application, on peut citer les solutions, en particulier les concentrés solubles dans l'eau, les concentrés émulsionnables, les émulsions, les suspensions concentrées, les aérosols, les poudres mouillables (ou poudre à pulvériser), les pâtes.

Les concentrés émulsionnables ou solubles comprennent le plus souvent 10 à 80 % de matière active, les émulsions ou solutions prêtes à l'application contenant, quant à elles, 0,01 à 20 % de matière active.

Ces compositions peuvent contenir aussi toute sorte d'autres ingrédients tels que, par exemple, des colloïdes protecteurs, des adhésifs, des épaississants, des agents thixotropes, des agents de pénétration, des stabilisants, des séquestrants, etc... ainsi que d'autres matières actives connues à propriétés pesticides (notamment insecticides ou fongicides) ou à propriétés favorisant la croissance des plantes (notamment des engrais) ou à propriétés régulatrices de la croissance des plantes. Plus généralement les composéa selon l'invention peuvent être associés à tous les additifs solides ou liquides correspondant aux techniques habituelles de la mise en formulation.

Par exemple, en plus du solvant, les concentrés émulsionnables peuvent contenir quand c'est nécessaire, 2 à 20 % d'additifs appropriés comme les stabilisants, les agents tensio-actifs, les agents de pénétration, les inhibiteurs de corrosion, les colorants ou les adhésifs précedemment cités.

Les doses d'emploi dans le cas d'une utilisation comme fongicides des composés selon l'invention peuvent varier dans de larges limites, notamment selon la virulence des champignons et les conditions climatiques.

D'une manière générale des compositions contenant 0,5 à 5000 ppm de substance active conviennent bien ; ces valeurs sont indiquées pour les compositions prêtes à l'application. Ppm signifie "partie par million". La zone de 0,5 à 5000 ppm correspond à une zone de $5x10^{-5}$ à 0,5 % (pourcentages pondéraux).

En ce qui concerne les compositions adaptées au stockage et au transport, elles contiennent plus avantageusement de 0,5 à 95 % (en poids) de substance active.

Ainsi donc, les compositions à usage agricole selon l'invention peuvent contenir les matières actives selon l'invention dans de très larges limites, allant de $5.10^{-5}$% à 95 % (en poids).

A titre d'exemple, voici la composition de quelques concentrés :

<u>Exemple F (formulation) 1</u>

| | |
|---|---|
| - matière active | 400 g/l |
| - dodécylbenzène sulfonate alcalin | 24 g/l |
| - nonylphénol oxyéthylé à 10 molécules d'oxyde d'éthylène | 16 g/l |
| - cyclohexanone | 200 g/l |
| - solvant aromatique | q.s.p 1 litre |

Selon une autre formule de concentré émulsionnable, on utilise :

<u>Exemple F 2 :</u>

| | |
|---|---|
| - matière active | 250 g |
| - huile végétale époxydée | 25 g |
| - mélange de sulfonate d'alcoylaryle et d'éther de polyglycol et d'alcools gras | 100 g |
| - diméthylformamide | 50 g |
| - xylène | 575 g |

A partir de ces concentrés, on peut obtenir par dilution avec de l'eau des émulsions de toute concentration désirée, qui conviennent particulièrement à l'application sur les feuilles.

Les suspensions concentrées, également applicables en pulvérisation, sont préparées de manière à obtenir un produit fluide stable ne se déposant pas et elles contiennent habituellement de 10 à 75 % de matière active, de 0,5 à 15 % d'agents tensioactifs, de 0,1 à 10 % d'agents thixotropes, de 0 à 10 % d'additifs appropriés, comme des anti-mousses, des inhibiteurs de corrosion, des stabilisants, des agents de pénétration et des adhésifs et, comme support, de l'eau ou un liquide organique dans lequel la matière active est peu ou pas soluble : certaines matières solides organiques ou des sels minéraux peuvent être dissous dans le support pour aider à empêcher la sédimentation ou comme antigels pour l'eau.

Les poudres mouillables (ou poudre à pulvériser) sont habituellement préparées de manière qu'elles contiennent 5 à 95 % de matière active, et elles contiennent habituellement, en plus du support solide, de 0 à 5 % d'un agent mouillant, de 3 à 10 % d'un agent dispersant, et, quand c'est nécessaire, de 0 à 10 % d'un ou plusieurs stabilisants et/ou autres additifs, comme des agents de pénétration, des adhésifs, ou des agents antimottants, colorants, etc...

A titre d'exemple, voici diverses compositions de poudres mouillables :

Exemple F 3 :

| | |
|---|---|
| - matière active | 50 % |
| - lignosulfonate de calcium (défloculant) | 5 % |
| - isopropylnaphtalène sulfonate (agent mouillant anionique) | 1 % |
| - silice antimottante | 5 % |
| - kaolin (charge) | 39 % |

Une autre composition de poudre à pulvériser à 70 % utilise les constituants suivants :

Exemple F 4 :

| | |
|---|---|
| - matière active | 700 g |
| - dibutylnaphtylsulfonate de sodium | 50 g |
| - produit de condensation en proportions 3/2/1 d'acide naphtalène sulfonique, d'acide phénolsulfonique et de formaldéhyde | 30 g |
| - kaolin | 100 g |
| - craie de champagne | 120 g |

Une autre composition de poudre à pulvériser à 40 % utilise les constituants suivants :

Exemple F 5 :

| | |
|---|---|
| - matière active | 400 g |
| - lignosulfonate de sodium | 50 g |
| - dibutylnaphtalène sulfonate de sodium | 10 g |
| - silice | 540 g |

Une autre composition de poudre à pulvériser à 25 % utilise les constituants suivants :

Exemple F 6 :

| | |
|---|---|
| - matière active | 250 g |
| - lignosulfonate de calcium | 45 g |
| - mélange équipondéral de craie de Champagne et d'hydroxyéthylcellulose | 19 g |
| - dibutylnaphtalène sulfonate de sodium | 15 g |
| - silice | 195 g |
| - craie de Champagne | 195 g |
| - kaolin | 281 g |

Une autre composition de poudre à pulvériser à 25 % utilise les constituants suivants :

Exemple F 7 :

| | |
|---|---|
| - matière active | 250 g |
| - isooctylphénoxy-polyoxyéthylène-éthanol | 25 g |
| - mélange équipondéral de craie de Champagne et d'hydroxyéthylcellulose | 17 g |
| - aluminosilicate de sodium | 543 g |
| - kieselguhr | 165 g |

Une autre composition de poudre à pulvériser à 10 % utilise les constituants suivants :

Exemple F 8 :

| | |
|---|---|
| - matière active | 100 g |
| - mélange de sels de sodium de sulfates d'acides gras saturés | 30 g |
| - produit de condensation d'acide naphtalène sulfonique et de formaldéhyde | 50 g |
| - kaolin | 820 g |

Pour obtenir ces poudres à pulvériser ou poudres mouillables, on mélange intimement les matières actives dans des mélangeurs appropriés avec les substances additionnelles et on broie avec des moulins ou autres broyeurs appropriés. On obtient par là des poudres à pulvériser dont la mouillabilité et la mise en suspension sont avantageuses ; on peut les mettre en suspension avec de l'eau à toute concentration désirée et ces suspensions sont utilisables très avantageusement en particulier pour l'application sur les feuilles ou semences des végétaux.

A la place des poudres mouillables, on peut réaliser des pâtes. Les conditions et modalités de réalisation et d'utilisation de ces pâtes sont semblables à celles des poudres mouillables ou poudres à pulvériser.

Comme cela a déjà été dit, les dispersions et émulsions aqueuses, par exemple les compositions obtenues en diluant à l'aide d'eau une poudre mouillable ou un concentré émulsionnable selon l'invention, sont comprises dans le cadre général de la présente invention. Les émulsions peuvent être du type eau-dans-l'huile ou huile-dans-l'eau et elles peuvent avoir une consistance épaisse comme celle d'une "mayonnaise".

Les granulés destinés à être disposés sur le sol sont habituellement préparés de manière qu'ils aient des dimensions comprises entre 0,1 et 2 mm et ils peuvent être fabriqués par agglomération ou imprégnation. En général, les granulés contiennent 0,5 à 25 % de matière active et 0 à 10 % d'additifs comme des stabilisants, des agents de modification à libération lente, des liants et des solvants.

Selon un exemple de composition de granulé, on utilise les constituants suivants :

Exemple F 9 :

| | |
|---|---|
| - matière active | 50 g |
| - épichlorhydrine | 2,5 g |
| - éther de cétyle et de polyglycol | 2,5 g |
| - polyéthylène glycol | 35 g |
| - kaolin (granulométrie : 0,3 à 0,8 mm) | 910 g. |

Dans ce cas particulier on mélange la matière active avec l'épichlorhydrine et on dissout avec 60 g d'acétone ; on ajoute alors le polyéthylène glycol et l'éther de cétyle et de polyglycol. On arrose le kaolin avec la solution obtenue et on évapore ensuite l'acétone sous vide. On utilise avantageusement un tel microgranulé pour lutter contre les champignons du sol.

Les composés de formule (I) peuvent encore être utilisés sous forme de poudres pour poudrage ; on peut aussi utiliser une composition comprenant 50 g de matière active et 950 g de talc ; on peut aussi utiliser une composition comprenant 20 g de matière active, 10 g de silice finement divisée et 970 g de talc ; on mélange et broie ces constituants et on applique le mélange par poudrage.

Les exemples I à VII illustrent des modes particuliers de préparation de composés selon l'invention ainsi que ces composés eux-mêmes. La nomenclature des composés a été indiquée selon les normes françaises mis à part le fait que la numérotation des substituants a été mise avant les substituants eux-mêmes.

Exemple I : Préparation du 1-[2-(2,4-dichlorophényl) 2-hydroxy 4,5-dibromo pentyl] 1 H 1,2,4-triazole

Etape a) Préparation du 1-chloro 2-(2,4-dichlorophényl) 4-pentène 2-ol:

Un dérivé organomagnésien est préparé par addition d'une solution de bromure d'allyle (180 ml) dans le tétrahydrofuranne (200 ml) à une suspension de magnésium (126 g) dans le tétrahydrofuranne (400ml) à 10° C.

On additionne à -10° C une solution de trichloro-alpha, 2, 4 acétophénone (232 g) dans le tétrahydrofuranne (250 ml), neutralise à l'acide acétique. On lave à l'eau, séche, évapore. Une huile incolore est obtenue (258 g). Point d'ébullition (3 $10^{-2}$mmHg) = 140-142° C.

Etape b) Préparation du 1-[2-(2,4-dichlorophényl)2-hydroxy 4-pentènyl] 1 H 1,2,4-triazole

Un mélange de produit obtenu à l'étape a) (106 g), de triazole (55 g) et de carbonate de potassium (160 g) est chauffé pendant quatre heures à 120° dans le diméthylformamide (600 ml). Les insolubles sont filtrés, lavés au diméthylformamide et le mélange réactionnel est concentré sous vide. Le résidu, dissous dans le chlorure de méthylène, est lavé à l'eau puis concentré. Le produit est obtenu par cristallisation dans l'acétate d'éthyle après dilution à l'heptane. Un solide rose pâle (97 g) est isolé fondant à 101°.

Etape c) Préparation du composé n° 1

Le composé obtenu à l'étape b (35 g) dans le chloroforme (200 ml) est traité à 0° par du brome. Après décoloration, le solvant est évaporé. On obtient un solide ayant un point de fusion de 116° C.

Exemple II - Préparation de 1-[2-(2,4-dichlorophényl) 2-hydroxy 4,5-dichloro pentyl] 1 H 1,2,4-triazole. Composé n° 2.

La chlorhydrine (113 g) obtenue à l'étape a de l'exemple I en solution dans le DMF (100 ml) est coulée dans un mélange de $K_2CO_3$ (207 g) et de 1,2,4-triazole (35,9g)dans le DMF (900 ml) à 125° C. Après chauffage à 125° C, filtration des insolubles et évaporation, lavage, recristallisation, on obtient le dérivé triazole IId (103g) sous forme d'un solide brun clair.

Ce dérivé triazole, dissous dans HCl aqueux concentré, est traité par du chlore jusqu'à persistance de la couleur jaune. Le mélange est laissé ensuite à température ambiante puis dilué. Le précipité formé est filtré et traité par le bicarbonate de sodium aqueux. Après extraction, séchage, évaporation et cristallisation, on obtient un solide blanc. point de fusion 126° C.

De la même manière on obtient les 1-[2-(4-chlorophényl) 2-hydroxy 4,5-dichloro pentyl] 1 H 1,2,4-triazole. Composé n° 3 P.F 82° C.

1-[2-(2,4-dichlorophényl) 2-hydroxy 3-méthyl 4,5 dibromopentyl] 1H 1, 2, 4-triazole. Composé n° 4. Huile.

1-[2-(2,4-dichlorophényl) 2-hydroxy 3-méthyl 4,5 dichloropentyl] 1, 2, 4-triazole. Composé n° 5. Huile.

Les exemples III à V illustrent les applications fongicides des composés selon l'invention.

dans ces exemples, les pulvérisations de solutions ou suspensions de matières actives sont effectuées dans des conditions telles que la pulvérisation d'une solution ou suspension de concentration égale à 1 g/l correspond en moyenne à l'application d'environ 2 microgrammes de matière active par $cm^2$ de feuille de la plante.

On prépare par broyage fin une émulsion aqueuse de la matière active à tester ayant la composition suivante :

- matière active à tester : 60 mg
- Tween 80 (agent tensio actif) constitué d'un oléate de dérivé polyéxyéthylèné du sorbitan) dilué à 10 % dans

l'eau : 0,3 ml
- on complète à 60 ml d'eau.

Cette émulsion aqueuse est ensuite diluée par de l'eau pour obtenir la concentration désirée.

Dans ces exemples on considère qu'un produit exerce une protection totale vis à vis d'une maladie fongique lorsque la protection est d'au moins 95 % .

Exemple III - Test in vivo sur "Piricularia oryzae" responsable de la Piriculariose du riz (Rice Blast)

Du riz, en godets, semé dans un mélange 50/50 de tourbe enrichie et de pouzzolane, est traité au stade 10 cm de hauteur par pulvérisation d'une émulsion aqueuse (appelée bouillie) ci-dessus définie. L'essai est répété deux fois. Au bout de 24 heures, on inocule par application sur les feuilles, d'une suspension de spores obtenues en culture pure (500 spores / ml).

Les plants contaminés sont placés en incubation à 25° C et 100% d'humidité relative pendant 24h avant d'être placés en cellule d'observation.

La lecture se fait 8 jours après la contamination. Dans ces conditions, on observe à 1g/l, une protection totale pour les composés 1, 2, 3, 4, 5.

Exemple IV - Test in vivo sur Erysiphe graminis sur orge (oïdium de l'orge)

De l'orge, en godets, semée dans un mélange 50/50 de tourbe enrichie et de pouzzolane est traitée au stade 10 cm de hauteur par pulvérisation d'une émulsion aqueuse (appelée bouillie) de concentration indiquée ci-dessus. L'essai est répété deux fois. Au bout de 24 heures, on saupoudre les plants d'orge avec des spores d'Erysiphe graminis, le saupoudrage étant effectué à l'aide de plants malades.

La lecture se fait 8 à 14 jours après la contamination.

Dans ces conditions, on observe les résultats suivants : à la dose de 1 g/l, protection totale avec les composés 1, 2, 3, 4, 5.

Exemple V - Test in vivo sur "Puccina recondita" responsable de la rouille du blé

Du blé, en godets, semé dans un mélange 50/50 de tourbe enrichie et de pouzzolane, est traité au stade 10 cm de hauteur par pulvérisation avec des émulsions aqueuses (appelée bouillie) décrite ci-dessus.

L'essai est répété deux fois.

Au bout de 24 heures, une suspension aqueuse de spores (50000 sp/$cm^3$) est pulvérisée sur le blé ; cette suspension a été obtenue à partir de plants contaminés. On place ensuite le blé pendant 48 heures en cellule d'incubation à environ 18° C et à 100 % d'humidité relative.

Au bout de ces 2 jours, le blé est tranporté en cellule d'observation où l'humidité relative est ramenée à 60 %. Le contrôle de l'état des plants se fait entre le 11ème et le 15ème jour après la contamination par comparaison avec le témoin non traité.

A la dose de 1 g/l, protection totale avec les composés 1, 2, 3, 4, 5.

Exemple 6 - Test in vitro sur champignons des semences et champignons du sol

On étudie l'action des composés selon l'invention sur les champignons suivants responsables des maladies des céréales et autres végétaux :

1) Botrytis cinerea sensible au carbendazime et aux imides cycliques
2) Botrytis cinerea résistant au carbendazime et aux imides cycliques
3) Fusarium oxysporum f.sp melonis
4) Rhizoctonia solani
5) Pseudocercosporella herpotrichoïdes
6) Septoria nodorum
7) Fusarium culmorum
8) Fusarium nivale
9) Fusarium roseum
10) Helminthosporium gramineum
11) Pyrenophora avenae
12) Helminthosporium teres
13) Rhizoctonia cerealis

Les chiffres figurant avant les noms seront utilisés pour identifier les champignons dans le tableau ci-dessous.

Pour chaque essai, on opère de la manière suivante : un milieu nutritif constitué de pomme de terre, de glucose et de gelose (milieu PDA) est introduit en surfusion dans une série de boîtes de Pétri (20 ml par boîte) après stérilisation à l'autoclave à 120° C.

Au cours du remplissage des boîtes, on injecte, dans le milieu en surfusion, une solution acétonique de la matière active, pour obtenir la concentration finale désirée.

On prend comme témoin des boites de Pétri analogues aux précédentes, dans lesquelles on coule des quantités similaires d'un milieu nutritif ne contenant pas de matière active.

Après 24 ou 48 h chaque boîte est ensemencée par dépôt d'un fragment de mycélium provenant d'une culture précédente du même champignon.

Les boîtes sont conservées pendant 2 à 10 jours (selon le champignon testé) à 22° C et on compare alors la

croissance du champignon dans les boîtes contenant la matière active à tester, à celle du même champignon dans la boîte utilisée comme témoin.

On détermine ainsi pour chaque composé testé le taux d'inhibition de la croissance du champignon considéré pour une dose de 30 ppm.

| Composé n° | Champignons | | | | | | |
|---|---|---|---|---|---|---|---|
| | 1 | 2 | 3 | 4 | 5 | 6 | 7 |
| 1 | 95 | 95 | 90 | 90 | 100 | 95 | 100 |
| 2 | 90 | 95 | 50 | 80 | 100 | 100 | 0 |
| 3 | 95 | 95 | 90 | 90 | 95 | 95 | 100 |
| 4 | 95 | 95 | 90 | 90 | 90 | 90 | 100 |
| 5 | 95 | 95 | 95 | 90 | 100 | 95 | 100 |
| Composé n° | Champignons | | | | | | |
| | 8 | 9 | 10 | 11 | 12 | 13 | |
| 1 | 95 | 80 | 95 | 100 | 95 | 100 | |
| 2 | 80 | 0 | 95 | 100 | 100 | 95 | |
| 3 | 100 | 100 | 95 | 100 | 100 | 95 | |
| 4 | 100 | - | - | 100 | 95 | 95 | |
| 5 | 100 | - | - | 100 | 100 | 95 | |

Il a également été trouvé que les buts précités pouvaient être atteints grâce à l'association d'un ou plusieurs composés de formule (I) avec au moins un fongicide du groupe (II) c'est-à-dire un fongicide choisi dans les sous-classes suivantes :

1. Les dérivés chlorés ou nitrés du benzène comme le quintozène ou le chlorothalonil,
2. Les dérivés dicarboximides comme le captane, le folpel, le captafol, l'iprodione, la procymidone,
3. Les dérivés comprenant un ou plusieurs hétérocycles comme les quinoléines, les morpholines,
4. Les dérivés de l'acide phosphoreux comme les phosphites métalliques,
5. Les dérivés de l'acide dithiocarbamique comme le manèbe ou le mancozèbe,
6. Les dérivés du phénol comme le dinocap,
7. Les dérivés des quinones comme le dithianon,
8. Les dérivés de l'acide carbamique et des benzimidazoles comme le carbendazime, le bénomyl, le thiophanate-méthyl,
9. Les dérivé soufrés,
10. Les amines et les amides telles que le dichloran, la carboxine, la triforine, le cymoxanil, le métalaxyl, l'ofurace,
11. Les diazines telles que le chinométhionate, le fénarinol, l'anilazine,
12. Les sulfamides telles que le dichlofluanide,
13. Les guanidines telles que la doguadine.
14. Les triazoles tels que ceux décrits dans le brevet britannique n°2046260 dont le contenu est incorporé par référence.

Les matières actives du groupe (II) sont des matières actives connues, la plupart d'entre elles étant décrites en détail dans des ouvrages tels que "The Pesticidal Manual" édité par "The British Crop Protection Council" dont la 7ème édition date de 1983.

Les associations selon l'invention sont le plus souvent de type binaire (une seule matière active du groupe II) mais on utilise aussi quelquefois des associations ternaires (2 matières actives du groupe II) ou quaternaire (3 matières actives du groupe II).

Parmi les matières actives du groupe II, on préfèrera encore les matières actives suivantes : chlorothalonil, iprodione, fenpropimorphe, tridémorphe, dinocap, dithianon, manèbe, mancozèbe, phoséthyl-Al, captane, carbendazime, captafol, soufre, diniconazole.

Les noms chimiques des produits énumérés ci-dessus sont indiqués au tableau annexé selon la nomenclature anglo-saxonne. C'est-à-dire avec l'indication de la position des substituants placée avant celui-ci.

Le rapport pondéral du composé selon l'invention avec les matières actives du groupe II décrite ci-dessus est de préférence compris entre 0,0003 et 3000 et avantageusement entre 0,001 et 1000.

Il est également possible d'associer avec les composés ou associations décrits ci-dessus des insecticides, des répulsifs, des régulateurs de germination.

TABLEAU DE NOMENCLATURE

| | |
|---|---|
| Chlorothalonil | Tétrachloro-isophtalonitrile |
| Iprodione | 3-(3,5-dichlorophényl)-N-isopropyl-2,4-dioxo imidazolidine-1-carboxamide |
| Fenpropimorphe | (+)-cis-4-[3-(4-tert-butylphenyl)-2-methyl propyl]-2,6-diméthylmorpholine |
| Tridémorphe | 2,6-diméthyl-4-tridécylmorpholine |
| Dinocap | 2-(1-méthylheptyl)-4,6-dinitrophényl crotonate |
| Dithianon | 5,10-dihydro-5,10-dioxonaphtol [2,3-b]-1,4-dithia-anthraquinone |
| Manèbe | Ethylène bis (dithiocarbamate) de manganèse |
| Mancozèbe | Complexe de manèbe avec un sel de zinc |
| Phoséthyl-Al | Tris-O-éthylphosphonate d'aluminium |
| Captane | N-(trichlorométhylthio) cyclohex-4-ene-1,2-dicarboximide |
| Carbendazime | Méthyl benzimidazol-2-yl carbamate |
| Captafol | N-(1,1,2,2-tétrachloroéthylthio) cyclohex-4-ene-1,2-dicarboximide |
| Diniconazol | 1-(2,4-dichlorophenyl)-4,4-dimethyl--2-(1,2,4-triazol-1-yl)-1-penten-3-ol. |

Ce dernier produit est décrit dans le brevet GB 2046260 déja mentionné.

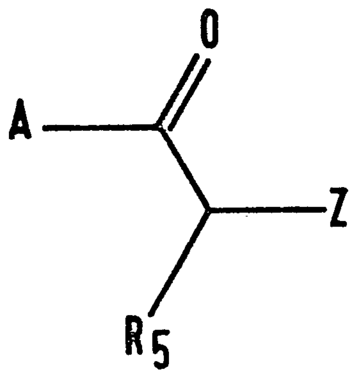

IIa

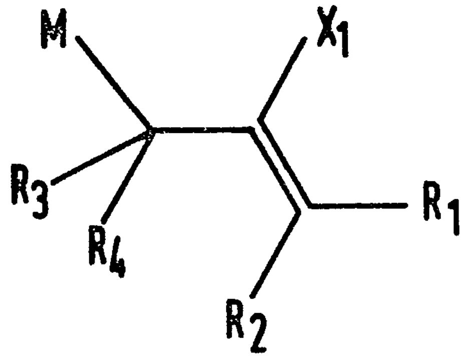

IIb

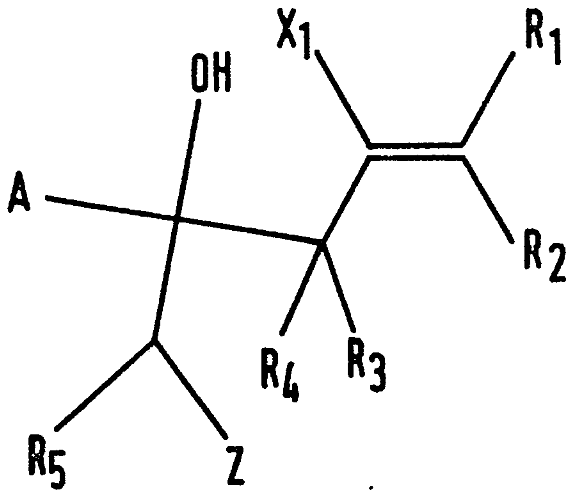

IIc

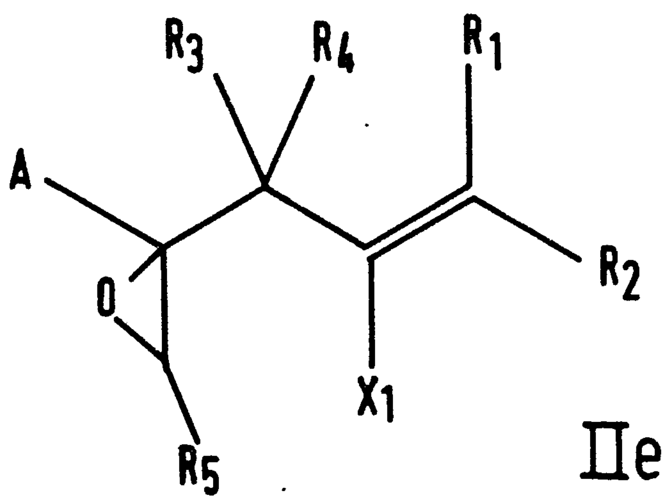

IIe

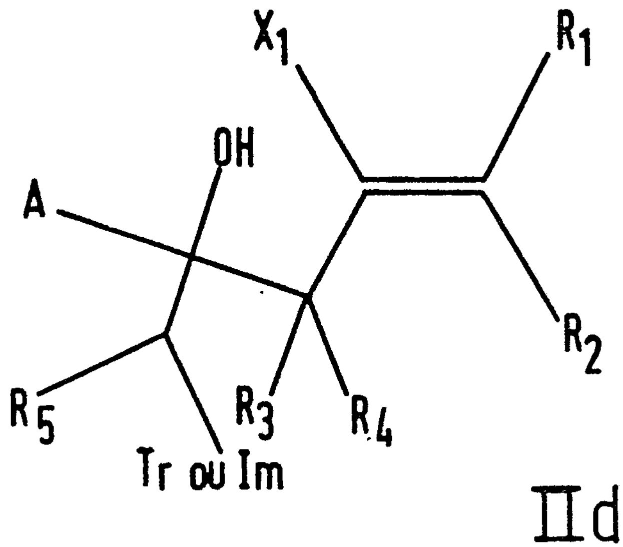

IId

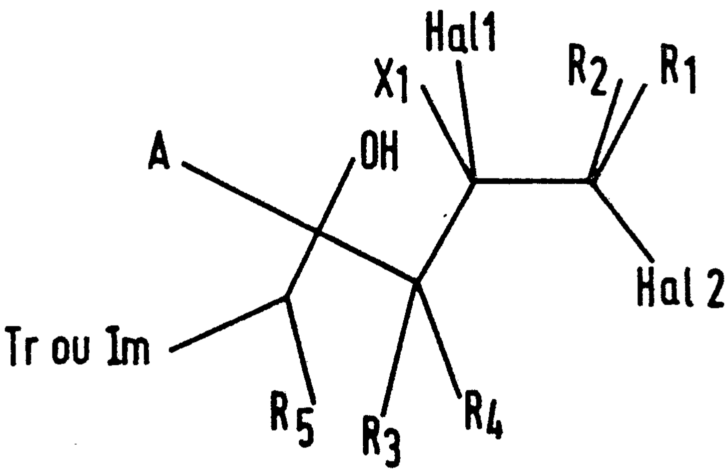

I

## Revendications

1) Composés à groupements triazole ou imidazole, de préférence triazole, caractérisés en ce qu'ils répondent à la formule générale I indiquée dans la description dans laquelle:A est le groupement (Y)nPh-(CH2)m où Ph est un noyau phénylène et m = 0 ou 1, de préférence 0,
Y est un atome d'halogène ou un radical cyano ou nitro, ou un groupe alkyle ou alkoxy, phényle, phénoxy, benzyle, benzyloxy, ces groupes étant éventuellement halogénés,
n est un nombre entier positif ou nul, inférieur à 6, les groupements Y pouvant étre identiques ou différents lorsque n est plus grand que 1,
Tr représente un groupe 1,2,4-triazole 1-yl, Im représente un groupe 1,3-imidazole 1-yl
$R_1$ à $R_5$, identiques ou différents, représentent l'atome d'hydrogène ou un radical alkyle inférieur, cycloalkyle inférieur, aryle (notamment phényle), aralkyle (notamment benzyle), alkoxy inférieur, alcanoyle inférieur, aroyle (notamment benzoyle), ces divers radicaux pouvant être éventuellement substitués (par exemple par un ou plusieurs atomes ou radicaux tels que les atomes d'halogènes, et les radicaux alkoxy inférieurs),
$X_1$ représente l'atome d'hydrogène, un atome d'halogène, un radical alkyle inférieur, un radical cycloalkyle inférieur, un radical aryle (notamment phényle), ou aralkyle (notamment benzyle), ces divers radicaux pouvant être éventuellement substitués de façon similaires aux groupes $R_1$ à $R_5$,
un groupement Q-$R_6$ dans lequel Q représente 0 ou S, et $R_6$ représente l'atome d'hydrogène ou un radical alkyle inférieur, un radical cycloalkyle inférieur, aryle (notamment phényle), aralkyle (notamment benzyle), acyle ou thioacyle (notamment acétyle, thioacétyle, propionyle, thiopropionyle), alkyloxythioyle, aryloxythioyle ou aralkyloxythioyle (le terme thioyle correspond à C = S (S)), ces radicaux pouvant être substitués de façon similaire aux groupes $R_1$ à $R_5$,
un groupement -N $R_8R_9$ dans lequel $R_8$ et $R_9$, identiques ou différents, représentent l'atome d'hydrogène ou un radical alkyle inférieur, un radical cycloalkyle inférieur, aryle (notamment phényle), aralkyle (notamment benzyle), ces divers radicaux pouvant être éventuellement substitués de façon similaire aux groupes $R_1$ à $R_5$, ou bien $R_8$ et $R_9$ peuvent former ensemble un radical unique divalent hydrocarboné, comprenant de 3 à 6 atomes de carbones, l'un de ces atomes de carbone pouvant être remplacé par un atome d'oxygène, de soufre ou d'azote, ledit radical unique divalent étant lui-même éventuellement substitué (par exemple par un ou plusieurs atomes d'halogène ou radicaux alkyle inférieur, alkoxy inférieur éventuellement halogénés, hydroxy),
Ha11, Ha12, identiques ou différents, représentent un atome d'halogène, de préférence choisis parmi le chlore ou le brome et avantageusement Ha11 et Ha12 sont identiques.
et leurs formes salifiées.

2) Composés selon la revendication 1 caractérisés en ce que Y est un atome d'halogène, de préférence le chlore, et n = 1, 2 ou 3.

3) Composés selon la revendication 2 caractérisés en ce que n = 1, ou 2 et Y est un atome d'halogène placé en ortho et/ou en para.

4) Composés selon la revendication 3 caractérisés en ce que les groupements $R_1$ à $R_5$, $X_1$ correspondent à un atome d'hydrogène, à un groupement alkyle inférieur, de préférence l'atome d'hydrogène, et/ou Ha11 et Ha12 correspondent tous les deux à l'atome de chlore.

5) Utilisation des composés selon l'une des revendications 1 à 4 à titre de fongicide.

6) Compositions fongicides, caractérisées en ce qu'elles contiennent comme matière active un composé selon l'une des revendications 1 à 4, cette matière active étant en association avec au moins un support inerte, acceptable en agriculture.

7) Compositions selon la revendication 6, caractérisées en ce qu'elles contiennent 0,5 à 95 % de matière active.

8) Procédé pour lutter contre les maladies fongiques des cultures, caractérisé en ce qu'on applique une dose efficace d'une matière active selon l'une des revendications 1 à 4, la matière active étant de préférence appliquée à raison de 0,005 à 5 kg/ha, de préférence de 0,01 à 0,5 kg/ha..

9) Procédé pour lutter contre les piricularioses, les oidiums, les rouilles des cultures, caractérisé en ce qu'on applique une dose efficace d'une matière active selon l'une des revendications 1 à 4, la matière active étant de préférence appliquée à raison de 0,005 à 5 kg/ha, de préférence de 0,01 à 0,5 kg/ha..

Office européen des brevets

# RAPPORT DE RECHERCHE EUROPEENNE

Numero de la demande

EP 88 42 0366

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | EP-A-0 104 734 (PFIZER)<br>* Revendications *<br>--- | 1,5,6 | C 07 D 249/08<br>C 07 D 233/60<br>A 01 N 43/50<br>A 01 N 43/653 |
| A | EP-A-0 110 570 (PFIZER)<br>* Revendications *<br>----- | 1,5,6 | |
| | | | **DOMAINES TECHNIQUES RECHERCHES (Int. Cl.4)** |
| | | | C 07 D 249/00<br>C 07 D 233/00<br>A 01 N 43/00 |

Le présent rapport a été établi pour toutes les revendications

| Lieu de la recherche | Date d'achèvement de la recherche | Examinateur |
|---|---|---|
| LA HAYE | 03-02-1989 | CHOULY J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)